(19) 
Europäisches Patentamt
European Patent Office
Office européen des brevets

(11) **EP 4 289 351 A1**

(12) **EUROPEAN PATENT APPLICATION**
published in accordance with Art. 153(4) EPC

(43) Date of publication:
**13.12.2023 Bulletin 2023/50**

(21) Application number: **22836756.1**

(22) Date of filing: **27.06.2022**

(51) International Patent Classification (IPC):
*A61B 5/055* (2006.01)   *A61N 1/00* (2006.01)
*A61N 2/00* (2006.01)

(86) International application number:
**PCT/CN2022/101590**

(87) International publication number:
**WO 2023/280002 (12.01.2023 Gazette 2023/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.07.2021   CN 202110765832**

(71) Applicant: **Beijing Galaxy Circumference Technologies Co., Ltd.
Beijing 102206 (CN)**

(72) Inventors:
• **WEI, Kecheng
  Beijing 102206 (CN)**
• **ZHANG, Wei
  Beijing 102206 (CN)**
• **ZHANG, Qiong
  Beijing 102206 (CN)**

(74) Representative: **Isarpatent
Patent- und Rechtsanwälte
Barth Hassa Peckmann & Partner mbB
Friedrichstraße 31
80801 München (DE)**

(54) **TARGET DETERMINATION METHOD AND APPARATUS, ELECTRONIC DEVICE, STORAGE MEDIUM, AND NEURO REGULATION DEVICE**

(57)     The present disclosure provides a target identification method, a target identification device, an electronic apparatus, a storage medium and a neuromodulation apparatus. The target identification method includes: acquiring scan data of a subject, the scan data including data obtained from magnetic resonance imaging of the subject's brain; determining the corresponding relationship of regions of interest of a disease type of the subject according to the disease type of the subject; determining at least one disease region of interest and at least one target region of interest of the subject in the scan data according to the corresponding relationship of the regions of interest; determining connectivity between each of voxels in the target region of interest and each disease region of interest in the at least one disease region of interest; and determining the positions of the voxels with the connectivity meeting a preset target connectivity condition in the target region of interest as the targets.

FIG. 2

EP 4 289 351 A1

**Description**

**Background of the Disclosure**

**Technical Field**

The present disclosure relates to the field of computer technology, and in particular, to a target identification method and device, an electronic apparatus, a storage medium, and a neuromodulation apparatus.

**Brief Description of the Related Art**

How to select the neuromodulation target in the human brain is a technical problem in the current neuroscience field. And an effective auxiliary means for helping to screen the personalized neuromodulation targets is urgently needed at present.

**Summary of the Disclosure**

The disclosure provides a target identification method, a target identification device, an electronic apparatus, a storage medium and a neuromodulation apparatus, which are used for screening the personalized neuromodulation targets.

In a first aspect, the present disclosure provides a target identification method, including: acquiring scan data of a subject, the scan data including data obtained from magnetic resonance imaging of the subject's brain; determining at least two regions of interest (ROI) of the subject from the scan data; determining corresponding relationship of regions of interest of a disease type of the subject according to the disease type of the subject; determining at least one disease region of interest(DROI) and at least one target region of interest(TROI) in the at least two regions of interest according to the corresponding relationship of the regions of interest; determining a connectivity of each of voxels in the target region of interest with each of the at least one disease region of interest; and determining positions of the voxels with the connectivities meeting a preset target connectivity condition in the target region of interest as the targets.

In some exemplified embodiments, the determining the connectivity of each of voxels in the target region of interest with each of the at least one disease region of interest includes:
determining connectivity coefficients of each of voxels in the target region of interest with each disease region of interest in the at least one disease region of interest as the connectivity between corresponding voxels in the target region of interest and corresponding disease region of interest in the at least one disease region of interest.

In some exemplified embodiments, the determining positions of the voxels with the connectivity meeting the preset target connectivity condition in the target region of interest as the targets includes:
determining the positions of the voxels with absolute values of the connectivity greater than a preset target connectivity threshold in the target region of interest as the targets.

In some exemplified embodiments, the determining positions of the voxels with the connectivity meeting the preset target connectivity condition in the target region of interest as the targets includes:

sorting voxels in the target region of interest in descending order by the absolute values of the connectivity to obtain a voxel sequence; and
determining positions of voxels with rankings from a first one to a preset rank number in the voxel sequence as the targets.

In some exemplified embodiments, the preset rank number includes a product of the total number of voxels in the target region of interest and a preset proportion value.

In some exemplified embodiments, the determining at least two regions of interest of the subject from the scan data includes:

determining the connectivity between every two voxels in the scan data to produce a brain connectivity matrix corresponding to the scan data; and
forming the at least two regions of interest based on a brain area template of a standard brain and the brain connectivity matrix.

In some exemplified embodiments, the determining at least two regions of interest of the subject from the scan data includes:

determining the connectivity between every two voxels in the scan data;

dividing the brain anatomy of the subject corresponding to the scan data into a plurality of large regions , and subdividing each of the plurality of large regions into a plurality of brain areas, wherein each of the plurality of brain areas includes at least one voxel; and

combining brain areas with the connectivity of voxels among the plurality of brain areas higher than a preset brain area voxel connectivity threshold and thus forming the at least two regions of interest.

In some exemplified embodiments, the magnetic resonance imaging includes: structural magnetic resonance imaging, and/or task state functional magnetic resonance imaging, and/or resting state functional magnetic resonance imaging.

In a second aspect, the present disclosure provides a target identification device, including:

a data acquisition unit configured to acquire scan data of a subject, the scan data including data obtained from magnetic resonance imaging of the subject's brain;

a processing unit configured to determine at least two regions of interest of the subject from the scan data;

a setting unit configured to determine corresponding relationship of region of interest of a disease type of the subject according to the disease type and to determine at least one disease region of interest and at least one target region of interest in the at least two regions of interest according to the corresponding relationship of region of interest; and

a target identification unit,

wherein the processing unit is further configured to determine a connectivity of voxels in a target region of interest with each of the at least one disease region of interest; and

the target identification unit is configured to determine voxels in the target region of interest with the connectivity meeting a preset target connectivity condition, as the targets.

In some exemplified embodiments, the processing unit is further configured to:

determine each disease region of interest in the at least one disease region of interest;

determine connectivity coefficients of voxels in the target region of interest to each disease region of interest in the at least one disease region of interest as the connectivity between corresponding voxels in the target region of interest and corresponding disease region of interest in the at least one disease region of interest.

In some exemplified embodiments, the target identification unit is further configured to:
determine the positions of the voxels with absolute values of the connectivity greater than a preset target connectivity threshold in the target region of interest as the targets.

In some exemplified embodiments, the target identification unit is further configured to:

sort voxels in the target region of interest in descending order by the absolute values of the connectivity to obtain a voxel sequence; and

determine positions of voxels with rankings from a first one to a preset rank number in the voxel sequence as the targets.

In some exemplified embodiments, the preset rank number includes a product of the total number of voxels in the target region of interest and a preset proportion value.

In some exemplified embodiments, the processing unit is further configured to:

determine the connectivity between every two voxels in the scan data to produce a brain connectivity matrix corresponding to the scan data; and

form the at least two regions of interest based on a brain area template of a standard brain and the brain connectivity matrix.

In some exemplified embodiments, the processing unit is further configured to:

determine the connectivity between every two voxels in the scan data;

divide the brain anatomy of the subject corresponding to the scan data into a plurality of large regions, and subdivide each of the plurality of large regions into a plurality of brain areas, wherein each of the plurality of brain areas includes at least one voxel; and

combine brain areas with the connectivity of voxels among the plurality of brain areas higher than a preset brain area voxel connectivity threshold and form the at least two regions of interest.

In some exemplified embodiments, the magnetic resonance imaging includes: structural magnetic resonance imaging, and/or task state functional magnetic resonance imaging, and/or resting state functional magnetic resonance imaging.

In a third aspect, the present disclosure provides an electronic apparatus, including:

at least one processor; and
a storage device having at least one program stored thereon,
wherein the at least one program, when executed by the at least one processor, causes the at least one processor to execute the method according to any one of the above embodiments.

In a fourth aspect, the present disclosure provides a computer readable storage medium having a computer program stored thereon, wherein the computer program, when executed by at least one processor, execute the method according to any of the above embodiments.

In a fifth aspect, the present disclosure provides a neuromodulation apparatus configured to make neuromodulation on a target of a subject in accordance with a preset neuromodulation solution; wherein the target is determined by the method according to any one of the above embodiments.

In some exemplified embodiments, the preset neuromodulation solution includes at least one of:

deep brain electrical stimulation;
transcranial electrical stimulation;
electroconvulsive therapy;
electrical stimulation based on cortical brain electrodes;
transcranial magnetic stimulation;
focused ultrasound neuromodulation;
magnetic resonance guided high-intensity focused ultrasound therapy neuromodulation; and photobiomodulation therapy.

In order to achieve the identification of neuromodulation targets, the current common technical means includes:

1. Determining neuromodulation targets based on group-level task-state functional magnetic resonance imaging (fMRI). However, the drawbacks of this technical means include: the task state fMRI has low signal-to-noise ratio and low repeatability, and requires a subject to have certain cognitive level, and the functional area result of the task-state fMRI greatly depends on task design greatly, and the difficulty in determining the baseline level of a functional area is great.

2. Determining neuromodulation targets by finding an approximate location of a specific functional area on the surface of a patient's scalp for projection on body surface by means of clinical experience based on brain anatomy, such as the left dorsal lateral prefrontal cortex (DLPFC) localization method (often referred to as the "5 cm" localization method) approved by the U.S. food And Drug administration (FDA) for repetitive transcranial magnetic stimulation (rTMS) to treat treatment-resistant depression. The drawbacks of this technical means include: the individual anatomical structure difference is ignored, and the positioning precision is low, so that the positioning of the neuromodulation targets is not accurate; and individual functional network differences are ignored, and the positions of the targets may be located in other brain functional areas.

3. Determining neuromodulation targets according to the electrode cap, such as an international 10-20 electrode cap positioning method. The drawbacks of this technical means include: the individual anatomical structure difference is ignored, and the positioning precision is low, so that the positioning of the neuromodulation targets is not accurate; and individual functional network differences are ignored.

4. Determining neuromodulation targets based on an ROI defined by an anatomical structure or population-averaged fMRI study. The drawbacks of this technical means include: various nerve and mental diseases often have no explicit focus of disease, but only show abnormal functions of a nervous system, and have the disease characteristics that a simple anatomical structure cannot reflect; and the etiology of neurological and psychiatric diseases is complex, and depends on individual differences, thus treatment regimens based on population-averaged fMRI are typically inefficient.

5. Determining neuromodulation targets according to the tissue structure metabolism condition reflected by the PET scan data. The drawbacks of this technical means include: PET scanning is expensive, thus placing an increased medical burden; certain radiation exists in the scanning process; PET scanning is limited in the use of neurological and psychiatric diseases; and the image signal to noise ratio is low, the accuracy of determining the targets is influenced by unclear boundaries of anatomical structures, and the clinical treatment effectiveness is low.

The target identification method, the target identification device, the electronic apparatus, the storage medium and the neuromodulation apparatus provided by the disclosure are realized by acquiring scan data of a subject, wherein the scan data includes data obtained by performing magnetic resonance imaging on the subject's brain; determining at least two regions of interest of the subject from the scan data; determining corresponding relationship of the regions of interest of the disease type of the subject according to the disease type of the subject; determining at least one disease region of interest and at least one target region of interest of the subject in the at least two regions of interest according to the corresponding relationship of the regions of interest; determining a connectivity between voxels in the target region of interest and each of the at least one disease region of interest; and determining the positions of the voxels with the connectivity meeting the preset target connectivity condition in the target region of interest as the targets. The embodiment of the disclosure provides individual brain scan data by using functional magnetic resonance imaging, screens targets according to a functional connectivity mode of the regions of interest, and can effectively solve the problem of inaccurate neuromodulation targets caused by the fact that individual structure or functional differences are not considered in the traditional method on the basis of fully considering individual differences, thereby realizing the positioning of voxels corresponding to individualized neuromodulation targets.

**Brief Description of the Drawings:**

Various embodiments discussed herein will be generally shown in drawings by way of example, without limitation. Other features, objects, and advantages of the disclosure will become more apparent from following detailed description of non-limiting embodiments with reference to the drawings below.

FIG. 1 is an exemplary system architecture diagram in which an embodiment of the present disclosure may be applied;
FIG. 2 is a schematic flow chart of an embodiment of a target identification method according to the present disclosure;
FIG. 3 is a partially exploded schematic view of an embodiment of a step 202 of the target identification method shown in FIG. 2;
FIG. 4 is a partially exploded schematic view of another embodiment of the step 202 of the target identification method shown in FIG. 2;
FIG. 5 is a schematic diagram illustrating result of partitioning the functional brain network of a subject in practical application using the method in the above embodiment;
FIG. 6 is a schematic diagram showing a neuromodulation target located in the dorsolateral prefrontal lobe in practical application using the method in the above embodiment; FIG. 7 is a schematic block diagram of an embodiment of a target identification device according to the present disclosure; and
FIG. 8 is a schematic block diagram of a computer system suitable for use in implementing a terminal device or a server of the present disclosure.

**Detailed Description of the Preferred Embodiment**

In order that the characteristics and technical contents of the embodiments of the present disclosure can be understood in detail, a more particular description on how to implement the embodiments of the disclosure will be given by reference to the appended drawings. These drawings are only intended to illustrate, but are not intended to limit the embodiments of the disclosure.

In the description of the embodiments of the present disclosure, it should be noted that, unless otherwise specified and limited, the terms "connect", "connection", "connecting", "connected" should be understood broadly, for example, they may refer to an electrical connection or an internal communication between two elements, and they may include direct connection and indirect connection through an intermediate. The specific significations of these terms may be understood by those skilled in the art according to specific situations.

It should be noted that the terms "first\second\third" related to the embodiments of the present disclosure are only used to distinguish similar objects, but do not mean a specific ordering for the objects. And it should be understood that "first\ second \ third" may be interchanged in the specific order or sequence when allowed. It should be noted that the objects defined by "first\second\third" may be interchanged under appropriate circumstances such that embodiments of the disclosure described herein may be implemented in orders other than those illustrated or described herein.

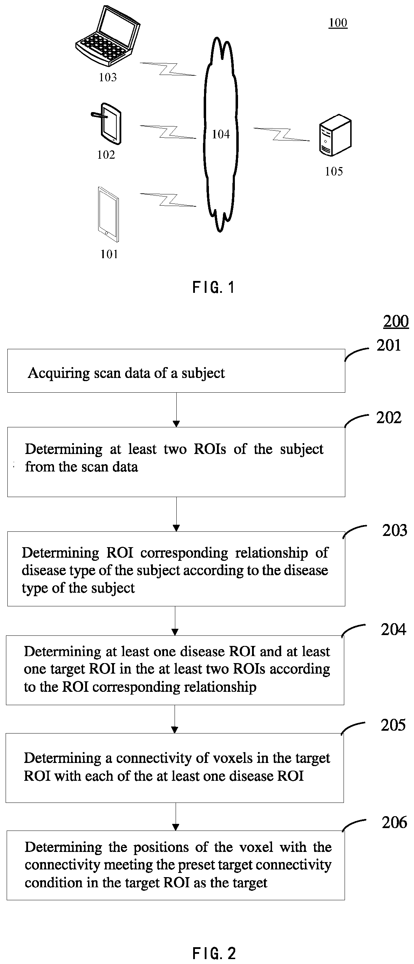

FIG. 1 illustrates an exemplary system architecture 100 for embodiments of the target identification methods or target identification devices of the present disclosure.

As shown in FIG. 1, the system architecture 100 can include terminal devices 101, 102, 103, a network 104, and a server 105. The network 104 is the medium used to provide communication links between the server 105 and each of the terminal devices 101, 102, 103. The network 104 may include various connection types, such as wired communication links, wireless communication links, or fiber optic cables, and so on.

Users may use terminal devices 101, 102, 103 to interact with the server 105 over the network 104 to receive or transmit messages or the like. Various communication client applications, such as a magnetic resonance imaging control application, a functional magnetic resonance imaging control application, a web browser application, a shopping application, a search application, an instant messaging tool, a mailbox client, social platform software, and the like, may be installed on the terminal devices 101, 102, and 103.

The terminal devices 101, 102, 103 can be implemented by hardware or software. When the terminal devices 101, 102, 103 are implemented by hardware, they may be various electronic devices with display screens, including but not limited to smart phones, tablet computers, laptop portable computers, desktop computers, and the like. When the terminal devices 101, 102, 103 are implemented by software, they can be installed in the above-listed multiple brain functional partition devices that electronically determine the subject. It may be implemented as a plurality of software or software modules (e.g. processes for providing brain functional atlas) or as a single software or software module. It is not particularly limited herein.

The server 105 can be a server that provides various services, such as a background data processing server that processes scan data transmitted by the terminal devices 101, 102, 103. The background data processing server can determine a plurality of brain functional partitions of the subject and voxels corresponding to each brain functional partition from the scan data and send them back to the terminal devices.

It should be noted that the server 105 can be implemented by hardware or software. When the server 105 is implemented by hardware, it may be implemented as a distributed server cluster composed of a plurality of servers, or may be implemented as a single server. When the server 105 is implemented by software, it may be implemented as multiple software or software modules (e.g., to provide distributed services), or as single software or software module. It is not particularly limited herein.

It should be noted that the target identification method provided by the present disclosure is generally performed by the server 105, and accordingly, the target identification device is generally disposed in the server 105.

It should be noted that, in some cases, the target identification method provided by the present disclosure may be executed by the server 105, by the terminal devices 101, 102, and 103, or both the server 105 and the terminal devices 101, 102, and 103. Accordingly, the target identification device may be disposed in the server 105, or may be disposed in the terminal devices 101, 102, and 103, or may be disposed partially in the server 105 or partially in the terminal devices 101, 102, and 103. And accordingly the system architecture 100 can include only the server 105, or only the terminal devices 101, 102, 103, or can include the terminal devices 101, 102, 103, the network 104 and the server 105. It is not particularly limited in the present disclosure.

It should be understood that the numbers of the terminal devices, the network, and the server in FIG. 1 is merely illustrative. There may be any number of terminal devices, networks, and servers, as desired for an implementation. Then, with reference to FIG. 2, a flow 200 of an embodiment of a target identification method according to the present disclosure is shown. The target identification method includes following steps:

At step 201, scan data of a subject is acquired.

In an embodiment of the present disclosure, the scan data includes data obtained by magnetic resonance imaging of the subject's brain.

The scan data includes a Blood Oxygen Level Dependency (BOLD) signal sequence corresponding to each of a predetermined number of voxels.

In this embodiment, an executor (e.g., the server shown in FIG. 1) of the target identification method may first acquire scan data of the subject locally or remotely from other electronic devices (e.g., the terminal devices shown in FIG. 1) connected to the executor through the network.

A voxel is short for a volumetric pixel. The voxel is conceptually similar to the smallest unit of a two-dimensional space, i.e., pixel, which is used in image data of a two-dimensional computer image. The voxel is the minimum unit of digital data on three-dimensional space segmentation, and is applied to the fields of three-dimensional imaging, scientific data, medical images and the like.

The BOLD signal sequence corresponding to the voxels means that the subject is scanned by magnetic resonance such that a BOLD signal is obtained for each of the voxels per preset time unit, and BOLD signals in a period are finally obtained, then the BOLD signals are arranged depending on order of the acquisition time to obtain a BOLD signal sequence corresponding to each of the voxels, where the number of the BOLD signals contained in the sequence can be an integer quotient obtained by dividing the time length corresponding to a target task by the preset time unit. For example, if the scan time duration is 300 seconds and the preset time unit is 2 seconds, there are 150 BOLD values in the BOLD signal sequence corresponding to each of the voxels, on other words, there are 150 frame data in the BOLD signal sequence corresponding to each of the voxels, or the BOLD signal sequence corresponding to each of the voxels is a vector with 150 dimensions, or the BOLD signal sequence corresponding to each of the voxels is a 1x150 matrix, which is not specifically limited by the present disclosure.

It is understood that the specific number of the voxels included in the scan data may be determined according to the scan accuracy of the functional magnetic resonance imaging or the magnetic resonance imaging, or may be determined according to the accuracy of the imaging apparatus, and the preset rank number is not limited to the specific number of voxels. In practical applications at present, the number of voxels in the subject's brain scan data is measured in tens of thousands or hundreds of thousands. With the progress of the scanning technology, the number of voxels included in the subject's brain scan data can be further increased.

In the present disclosure, the executor may obtain the scan data of the subject from other electronic devices (for example, the terminal devices shown in FIG. 1) connected locally or remotely to the executor through the network.

In an embodiment of the present disclosure, magnetic resonance imaging may include: structural magnetic resonance imaging, and/or task state functional magnetic resonance imaging, and/or resting state functional magnetic resonance imaging.

Data obtained by functional magnetic resonance imaging contain time sequence information, and correspond to a four-dimensional image. For example: sampling a functional magnetic resonance imaging image, sampling a 3-dimensional image matrix (Length * Width * Height, L*M * N), sampling 1 frame of data every 2 seconds, and sampling 150 frames of data within 6 minutes to form a functional magnetic resonance imaging data signal with L*M*N voxels * 150.

The data obtained by the structural magnetic resonance imaging are in form of a high-resolution three-dimensional gray scale anatomical structure image, such as T1w (T1 weighted imaging - highlighting tissue T1 relaxation (longitudinal relaxation) differences) and the related images thereof, T2w (T2 weighted imaging - highlighting tissue T2 relaxation (transverse relaxation) differences) and the related images thereof, a fluid attenuated inversion recovery (FLAIR) sequence and the related images thereof. The structural magnetic resonance imaging may also include magnetic resonance diffusion imaging, such as: diffusion-weighted imaging (DWI) and the related images thereof, diffusion tensor imaging (DTI) and the related images thereof, and the like.

DTI is a magnetic resonance technique used to study the anisotropy in diffusion of nerve tracts in central nervous system anatomy and to show white matter fibrous anatomy. DTI observes tissue microstructures by anisotropy in diffusion of water molecules in the tissue. The anisotropy of white brain matter is caused by parallel running myelin sheath axonal fibers, and the diffusion of white brain matter is the greatest in the direction of parallel nerve fibers, i.e. the fractional anisotropy (FA) of diffusion is the largest and can be approximately determined as 1 (actually, the fractional number can be greater than 0.9 and approximate to 1). The characteristic can reflect the space directionality of white brain matter by using a color mark, namely, the direction in which the diffusion is the fastest indicates the direction of fiber running. Imaging of the fiber bundle by DTI may produce a brain connectivity matrix that reflects the brain structure.

In an embodiment of the present disclosure, the functional magnetic resonance imaging may include: task state functional magnetic resonance imaging, and/or resting state functional magnetic resonance imaging.

It is understood that the resting state functional magnetic resonance imaging is the magnetic resonance imaging resulting from a magnetic resonance scan of of a subject's brain while the subject is not performing any tasks during the scan. The task state functional magnetic resonance imaging is the magnetic resonance imaging resulting from a magnetic resonance scan of the subject's brain while the subject performs a target task.

After acquiring the magnetic resonance scan data of the brain structure of the subject, various implementations can be used to determine atlas of the brain structure of the subject according to the magnetic resonance scan data of the brain structure of the subject, i.e., to determine which structural components are in the respective specific regions of the subject's brain. For example, it can be implemented using existing software for processing three-dimensional brain scan data, such as FreeSurfer which is magnetic resonance data processing software. For example, the deep learning model may be trained in advance based on a large amount of brain structure image scan sample data and labels of the corresponding brain structure component, and then the brain structure magnetic resonance scan data of the subject may be input into the trained deep learning model to obtain the corresponding atlas of the brain structure.

In some alternative embodiments, the executor pre-processes the scan data of the subject after acquiring the scan data.

In the present disclosure, the method of pre-processing is not particularly limited. As an example, the pre-processing may include:

preprocessing of images of the magnetic resonance imaging, for example,

(1) slice timing, realignment, temporal signal filtering, noise component regression, spatial smoothing, etc.;
(2) registering image of the functional magnetic resonance imaging with the structural image (if the structural image exists);
(3) projecting the functional magnetic resonance imaging signals onto the structural image (if the structural image exists), including the reconstructed images of the individual cerebral cortex or the associated group of average structural images.

The structural magnetic resonance imaging image is preprocessed (if the structural image exists), such as skull removal, field intensity correction, individual anatomical structure segmentation, cerebral cortex reconstruction and the like.

At step 202, at least two ROIs of the subject are determined from the scan data.

As regard to the above step 202, the present disclosure provides a plurality of alternative embodiments.

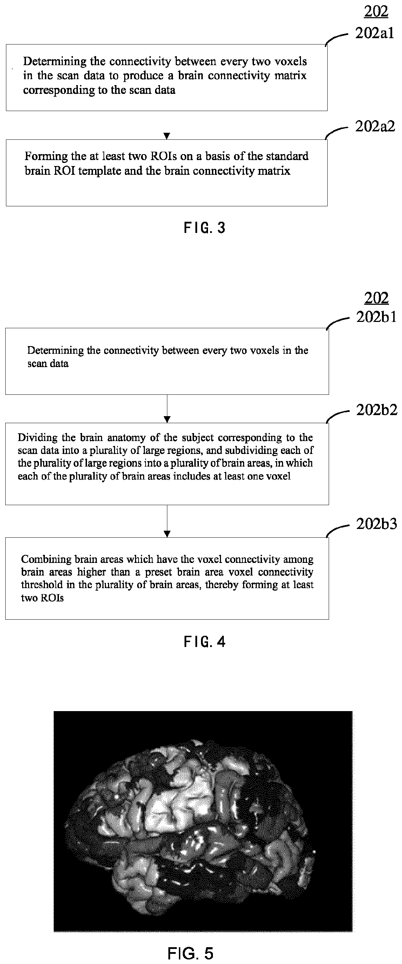

FIG. 3 is a partially exploded schematic view of an embodiment of the step 202 of the target identification method shown in FIG. 2. In some alternative embodiments, as shown in FIG. 3, the step 202 can specifically include: At step 202a1, the connectivity between every two voxels in the scan data is determined to produce a brain connectivity matrix corresponding to the scan data.

As an example, assuming that there are one hundred thousand voxels in the scan data and a BOLD signal sequence corresponding to each of the voxels includes T BOLD values, where T is a sampling number of a time dimension corresponding to a scan time, the brain connectivity matrix corresponding to the scan data is a 1 hundred thousand * 1 hundred thousand-order matrix, and the brain connectivity matrix can represent connectivity between every two voxels in the scan data. The connectivity between the two voxels may be calculated based on the T BOLD values corresponding to the voxels by using the Pearson correlation coefficient.

At step 202a2, at least two ROIs are formed on a basis of the standard brain ROI template and the brain connectivity matrix.

As an example, a brain functional network containing more than 2 ROIs can be established for a subject based on a standard brain ROI template using pattern recognition or machine learning methods. Methods may include, but are not limited to: Independent Component Correlation Algorithm (ICA), Principal Component Analysis (PCA), various types of clustering methods, factor Analysis, Linear Discriminant Analysis (LDA), various matrix decomposition methods, and the like. In the obtained brain functional network, there may be different positions of voxels in respective functional partitions for different tests, but each voxel is assigned to a specific ROI. That is, each ROI of the subject, may be a set of voxels made up of voxels in fMRI that have the same function.

FIG. 4 is a partially exploded schematic view of yet another embodiment of the step 202 of the target identification method shown in FIG. 2. In some alternative embodiments, as shown in FIG. 4, the step 202 can specifically include:

At step 202b1, the connectivity between every two voxels in the scan data is determined.

At step 202b2, the brain anatomy of the subject corresponding to the scan data are divided into a plurality of large regions, and each of the plurality of large regions is subdivided into a plurality of brain areas, in which each of the plurality of brain areas includes at least one voxel.

At step 202b3, brain areas, which have the voxel connectivity among brain areas higher than a preset brain area voxel connectivity threshold in the plurality of brain areas, are combined, thereby forming at least two ROIs.

As an example, a subject's brain is first divided into a plurality of large regions according to major anatomical boundaries; and then, each large region is divided by using functional connection, and the functional connection for each large region is determined according to the reliability (testretest reliability) of each large region. After each large region is divided, a plurality of brain areas are obtained, then the brain areas are combined according to the similarity of voxels contained in the brain areas, and the brain areas with high relative voxels are combined into one ROI. As an example, at least two ROIs can be finally determined in the whole brain.

In some exemplified embodiments, the step 202 can specifically include:

pre-selecting or generating a group brain functional atlas as a brain functional atlas template, and projecting the boundaries of at least two brain-brain areas in the brain functional atlas template to the brain scan data of the subject; and

adjusting the boundaries of the at least two brain-brain areas based on the brain scan data of the subject, thereby allowing boundaries of the adjusted brain-brain areas to match at least with the brain scan data of the subject and forming at least two ROIs.

As an example, the group brain functional atlases are directly projected to the subjects' brain, and then the boundaries of the brain areas projected by the group brain functional atlases are gradually adjusted according to the anatomical brain functional atlases of the subjects by means of a recursive algorithm until the boundaries of the brain areas tend to be stable. The recursive process will use the individual difference distribution of the connectivity of the subject, and the signal-to-noise ratio of the brain image of the subject itself, to determine the magnitude of the boundary adjustment of the brain-brain areas. Finally, the brain-brain areas are combined according to the correlation of voxels, thereby obtaining at least two ROIs.

In the present disclosure, the connectivity between the voxel and the ROI can include an average value of the connectivities of the voxel and each voxel in the ROI. The connectivity between two ROIs can include an average value of the connectivities between each voxel in one of the two ROIs and each voxel in the other ROI. The connectivity between the voxel and the brain area can include an average value of the connectivities between the voxel and each voxel in the brain area. The connectivity between two brain areas can include an average value of the connectivities between each voxel in one of the two brain areas and each of the voxels in the other brain area.

The connectivity characterizes the connection degree of brain connections and can also be expressed as a degree of correlation. Here, brain connections may include functional connections and structural connections. The functional connections can be obtained by calculating a Pearson correlation coefficient based on a BOLD time sequence corresponding to the voxels in the ROI; and structural connections include structural connections between ROIs such as obtained from fiber bundle imaging.

In some exemplified embodiments, the step 202 can specifically include:
establishing a standard ROI library, where the ROI library includes ROIs related to various test scenes and potential functional connection modes thereof. After a standard ROI library exists, corresponding ROIs can be obtained by screening of test scene types, and then at least two ROIs of the subject are obtained on the basis of them.

At step 203, ROI corresponding relationship of disease type of the subject is determined according to the disease type of the subject.

The disease type includes a disease type determined by a diagnosis of the subject or a disease type corresponding to a symptom to be treated in the subject.

The ROI corresponding relationship of the disease type can be inquired according to the existing ROI corresponding relationship of the determined disease type, and can also be set according to actual requirements, the manner in which the ROI for the type of disease is obtained is by way of example only and is not particularly limited.

At step 204, at least one disease ROI (DROI)and at least one target ROI(TROI) are determined in the at least two ROIs according to the ROI corresponding relationship.

For the disease types of the subjects, two types of ROI corresponding to the disease types of the subject are determined according to the preset ROI corresponding relationship of the disease type: (1) M disease ROIs; and (2) N target ROIs. M and N are both positive integers greater than or equal to 1.

It is understood that the target ROI is an ROI that includes a neural target corresponding to the disease ROI, and the neural target has a neural association with the desired brain functional partition, and can perform neural modulation on the desired brain functional partition through stimulation of the neural target.

At step 205, the connectivity of the voxels in the target ROI with each of the at least one disease ROI is determined.

In some exemplified embodiments, the step 205 can specifically include:
determining each of the at least one disease ROIs; the connectivity coefficients of the voxels in the target ROI with each of the at least one disease ROI are determined as the connectivity of the corresponding voxels in the target ROI with the corresponding disease ROI in the at least one disease ROI.

In the present disclosure, the connectivity coefficient is a pearson correlation coefficient, which is a coefficient used to measure a linear relationship between variables. The calculation formula is as follows:

$$\rho_{X,Y} = \frac{\text{cov}(X,Y)}{\sigma_X \sigma_Y} = \frac{E((X - \mu_X)(Y - \mu_Y))}{\sigma_X \sigma_Y} = \frac{E(XY) - E(X)E(Y)}{\sqrt{E(X^2) - E^2(X)}\sqrt{E(Y^2) - E^2(Y)}}$$

The formula is defined as: the pearson correlation coefficients ($\rho_{X,Y}$) of two consecutive variables (X, Y) are equal to the covariance cov (X, Y) between them divided by the product of their respective standard deviations ($\sigma_X$, $\sigma_Y$). Coefficients are always values between -1.0 and 1.0. If the coefficients are variables equal or approximately equal to 0, it will be uncorrelated. In contrast, if the coefficients are variables equal or approximately equal to 1 or -1, it will have strong correlation. Here, the term "approximately equal" may be understood as a difference from a target value within an allowable error range, 15for example, in the present disclosure, 0.01 may be approximately equal to 0, or 0.99 may be approximately equal to 1, which is merely illustrative. An allowable error range of approximately equal may be determined according to an accuracy required for calculation in practical applications.

At step 206, the position of the voxel with the connectivity meeting the preset target connectivity condition in the target ROI is determined as the target. The target can include coordinates corresponding to a single voxel, or can be a set of regions formed by some voxels.

In some exemplified embodiments, the step 206 can specifically include:
determining the position of the voxel with the absolute value of the connectivity greater than the preset target connectivity threshold of the target ROI as the target.

The preset target connectivity threshold may be set according to actual requirements. It only gives an example instead of having specific limitation on the preset target connectivity threshold. It should be understood that the larger the absolute value of the connectivity is, the greater the correlation between the voxels to be connected in the target ROI and the disease ROI will be. Therefore, the target of which the connectivity with the disease ROI meets the preset requirements, can be effectively screened by means of determining the voxel with the absolute value of the connectivity greater than the preset target connectivity threshold in the target ROI as the target.

In some exemplified embodiments, the step 206 can specifically include: sorting voxels in the target ROI in descending order by the absolute values of the connectivity to obtain a voxel sequence, and determining voxels with rankings from a first one to a preset rank number in the voxel sequence as the target.

The preset rank number may be set according to actual requirements. It only gives an example instead of having specific limitation on this. It can be understood that, in some application scenarios, the number of the targets obtained from the decision based on the preset target connectivity threshold in the above embodiments may be greater or less than the number of the targets needed for performing operations such as regulation and control on the targets in the subsequent stage, and therefore, the preset rank number may be set according to the number of the targets actually needed, so as to meet the number of the targets actually needed.

In some alternative embodiments, the preset rank number is a product of the total number of voxels in the target ROI and a predetermined proportion value.

The preset proportion value can be set according to actual requirements. For example, when the targets are subjected to operations such as regulation and control in the subsequent stage, in order to distinguish the targets conveniently in the operation, the number of the targets meeting the actual requirement proportion can be determined by setting the preset proportion value.

For a target ROI of the N target ROIs, the connectivity of each voxel in the target ROI with each target ROI of the M target ROIs (for example, the connection between the voxel and an average signal sequence of the target ROI corresponding to a test scene is calculated by using a Pearson correlation coefficient) is calculated, and then the voxels with rankings less than a predetermined rank value, or the voxels ranked in a predetermined top proportion of all of the voxels, or the voxels with the absolute value of the connectivity greater than a predetermined value, are selected as the targets from voxels in the target ROI. Here the connection involved may include a positive connection or a negative connection. For example, if the correlation coefficient between two voxels is between 0 and 1, the two voxels are connected positively, while the correlation coefficient is between -1 and 0, the two voxels are connected negatively. But in summary, the targets are found in the voxels corresponding to the target ROI.

The identification of targets according to the method is relatively accurate, and in practical application, scientific research personnel or medical care personnel can perform neuromodulation navigation on the subject according to the targets determined by the method by means of using optical navigation equipment or electromagnetic navigation equipment, and the effective rate of neuromodulation can be improved.

The present disclosure provides a neuromodulation apparatus configured for neuromodulating a target of a subject according to a predetermined neuromodulation solution. The target of the subject is determined according to the target identification method in any of the above embodiments of the present disclosure.

The neuromodulation apparatuses can include implantable neuromodulation apparatuses and non-implantable neuromodulation apparatuses, such as: event-related potential analysis systems, electroencephalography systems, brain-computer interface apparatuses. The present disclosure is not intended to limit the particular forms of neuromodulation apparatuses, which are presented herein by way of example only.

Regarding the neuromodulation of targets of the subject, the operator can connect the neuromodulation apparatus depending on the targets and then perform the neuromodulation operation, or the neuromodulation apparatus can perform the neuromodulation operation according to the targets of the subject input by the operator or acquired actively by the neuromodulation apparatus. This is merely an example instead of having a specific limitation on the neuromodulation of targets of a subject. The skilled person in the art may operate according to the actual utility of the neuromodulation apparatus. As an example, the preset neuromodulation solution may include, but is not limited to:

    a. neuromodulation solutions based on electrical pulse sequence

        i. deep brain electrical stimulation
        ii. transcranial electrical stimulation
        iii. electroconvulsive therapy
        iv. electrical stimulation based on cortical brain electrodes
        v. related derivatives of the above techniques

    b. neuromodulation solutions based on magnetic pulse sequence

        i. transcranial magnetic stimulation and related solutions
        ii. related derivatives of the above techniques

    c. ultrasound-based neuromodulation solutions

        i. focused ultrasound neuromodulation solutions

ii. magnetic resonance guided high-intensity focused ultrasound therapy system and related modulation solutions

iii. related derivatives of the above techniques

d. photobiomodulation therapy

i. photic stimulation in different wavelength and related solutions
ii. related derivatives of the above techniques

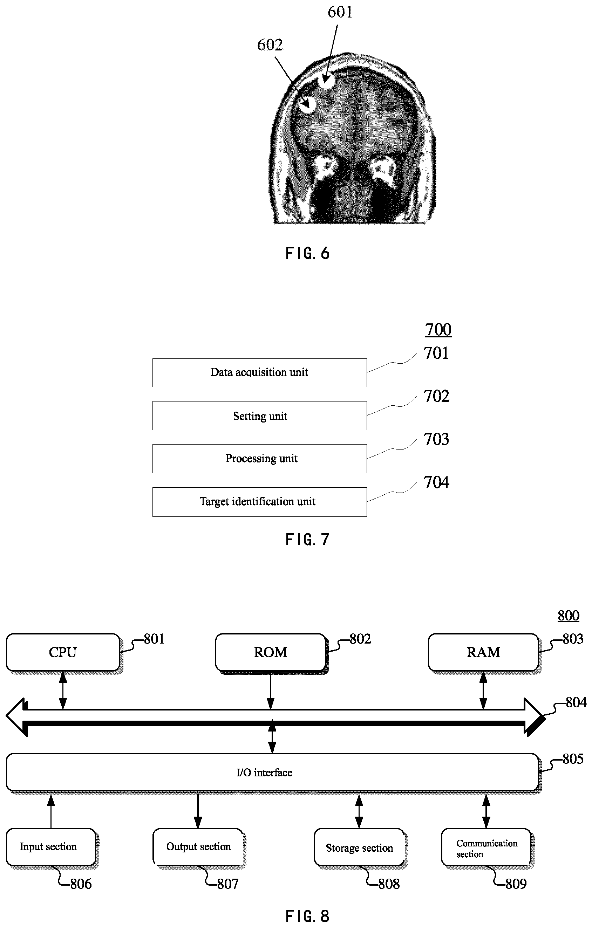

With the gradual development of novel neuromodulation apparatuses and neuromodulation technology, the target identification method in the present disclosure can be used to determine the targets of neuromodulation in future neuromodulation apparatuses and neuromodulation solutions, which also belongs to the protection scope of the present disclosure. To visually demonstrate the effect of the method in the above embodiments, as an example, FIG. 5 shows the result of partitioning the functional brain network of the subject by using the method in the above embodiment in practical application, and the different gray levels represent different functional partitions of the brain; and FIG. 6 shows the neuromodulation targets located in the dorsolateral prefrontal lobe using the method in the above embodiment in practical application. The first target 601 is a dorsal target and can be used for adjusting ROI corresponding to anxiety, and the second target 602 is a ventral target and can be used for adjusting ROI corresponding to depression.

The method for establishing the brain functional network in the present disclosure can efficiently and reliably acquire the function information of each part of the brain, and improve the accuracy of positioning brain areas. The functional positioning of the individual brain is assisted by the brain functional atlas of the group level, thereby improving the reliability of the result of positioning the neuromodulation targets.

With further reference to FIG. 7, as an implementation of the methods shown in the above figures, the present disclosure provides an embodiment of a target identification device, which corresponds to the embodiment of the method shown in FIG. 2, and the device can be applied in various electronic devices.

As shown in FIG. 7, a target identification device 700 of the present embodiment includes: a data acquisition unit 701, a setting unit 702, a processing unit 703 and a target identification unit 704.

The data acquisition unit 701 is configured to acquire scan data of a subject, wherein the scan data includes data from magnetic resonance imaging of the subject's brain; and the scan data includes a sequence of blood oxygen level dependent BOLD signals corresponding to each of a predetermined number of voxels.

The processing unit 703 is configured to determine at least two regions of interest, ROIs, of the subject from the scan data.

The setting unit 702 is configured to determine an ROI corresponding relationship of a disease type of the subject according to the disease type of the subject. And at least one disease ROI and at least one target ROI are determined among the at least two ROIs in accordance with the ROI corresponding relationship.

The processing unit 703 is further configured to determine a connectivity of voxels in the target ROI with each of the at least one disease ROI.

The target identification unit 704 is configured to determine a position of voxels in the target ROI with a connectivity meeting a preset target connectivity condition as a target.

In some exemplified embodiments, the processing unit 703 is further configured to:

determine each of the at least one disease ROI; and
determine the connectivity coefficients of the voxels in the target ROI with each of the at least one disease ROI as the connectivity of the corresponding voxels in the target ROI with the corresponding disease ROI in the at least one disease ROI.

In some exemplified embodiments, the target identification unit 704 is further configured to:
determine the position of the voxels with the absolute values of the connectivity greater than the preset target connectivity threshold of the target ROI as the target.

In some exemplified embodiments, the processing unit 703 is further configured to:

sort voxels in the target ROI in descending order by the absolute value of the connectivity to obtain a voxel sequence; and
determine positions of voxels with rankings from a first one to a preset rank number in the voxel sequence as targets.

In some exemplified embodiments, the predetermined number of voxels includes a product of the total number of voxels in the target ROI and a predetermined voxel proportion.

In some exemplified embodiments, the processing unit 703 is further configured to:

acquire a BOLD signal sequence corresponding to each of voxels in scan data;
determine the connection between every two voxels in the scan data based on the BOLD signal sequence corresponding to each of voxels to produce a brain connectivity matrix corresponding to the scan data; and
form at least two ROIs based on a brain area template of a standard brain and the brain connectivity matrix.

In some exemplified embodiments, the processing unit 703 is further configured to:

acquire a BOLD signal sequence corresponding to each of voxels in scan data;
determine the connection between every two voxels in the scan data based on the BOLD signal sequence corresponding to each of voxels;
divide the brain anatomical structure of the subject corresponding to the scan data into a plurality of large regions , and subdivide the plurality of large regions into a plurality of brain areas, wherein each of the plurality of brain areas includes at least one voxel; and
combine respective brain areas with the voxel connectivity higher than a preset brain area voxel connectivity threshold in the plurality of brain areas, thereby forming at least two ROIs.

In some exemplified embodiments, the functional magnetic resonance imaging includes: task state functional magnetic resonance imaging, and/or resting state functional magnetic resonance imaging.

It should be noted that, for implementation details and technical effects of each unit in the target identification device provided in the present disclosure, reference may be made to other embodiments in the present disclosure, and the details will be omitted herein.

Referring now to FIG. 8, it shows a schematic block diagram of a computer system 800 suitable for use in implementing a terminal device or server of the present disclosure. The terminal device or server shown in FIG. 8 is only an example, and should not bring any limitation to the function and the use range of the present disclosure.

As shown in FIG. 8, the computer system 800 includes a Central Processing Unit (CPU) 801 which can perform various appropriate actions and processes according to a program stored in a Read Only Memory (ROM) 802 or a program loaded from a storage section 808 into a Random Access Memory (RAM) 803. In the RAM 803, various programs and data necessary for the operation of the computer system 800 are also stored. The CPU 801, ROM 802, and RAM 803 are connected to each other via a bus 804. An Input/Output (I/O) interface 805 is also connected to the bus 804.

The following components are connected to the I/O interface 805: an input section 806 including a keyboard, a mouse, and the like; an output section 807 including such as a Cathode Ray Tube (CRT), a Liquid Crystal Display (LCD), and a speaker; a storage section 808 including a hard disk and the like; and a communication section 809 including a network interface card such as a LAN (Local Area Network) card, a modem. The communication section 809 performs communication processing via a network such as the Internet.

In particular, the processes described above with reference to the flow charts may be implemented as computer software programs, according to embodiments of the present disclosure. For example, embodiments of the present disclosure include a computer program product including a computer program carried on a computer-readable medium, the computer program including program code for performing the method illustrated by the flow chart. In such an embodiment, the computer program can be downloaded and installed from the network via communication section 809. The computer program performs the above-described functions defined in the method of the present disclosure when executed by the Central Processing Unit (CPU) 801. It should be noted that the computer readable medium of the present disclosure can be a computer readable signal medium or a computer readable storage medium or any combination of both. A computer readable storage medium may be, for example, but not limited to, an electronic, magnetic, optical, electromagnetic, infrared, or semiconductor system, apparatus, or device, or any combination of thereof. More specific examples of the computer readable storage medium may include, but are not limited to: an electrical connection having one or more wires, a portable computer disk, a hard disk, a Random Access Memory (RAM), a read-only memory (ROM), an erasable programmable read-only memory (EPROM or flash memory), an optical fiber, a portable compact disc read-only memory (CD-ROM), an optical storage device, a magnetic storage device, or any suitable combination of thereof. In the present disclosure, the computer readable storage medium may be any tangible medium that can contain, or store a program for use by or in connection with an instruction execution system, apparatus, or device. In contrast, in the present disclosure, a computer-readable signal medium may include a propagated data signal carried with computer-readable program code therein in baseband or as part of a carrier wave. Such a propagated data signal may take any of a variety of forms, including, but not limited to, electro-magnetic signals, optical signals, or any suitable combination thereof. The computer readable signal medium may be any computer readable medium that is not a computer readable storage medium and that can communicate, propagate, or transmit a program for use by or in connection with an instruction execution system, apparatus, or device. Program code embodied on a computer readable medium may be transmitted using any appropriate medium, including but not limited to: wireless, wire, fiber optic cable, RF, etc., or any suitable combination of thereof.

Computer program code for carrying out operations for the present disclosure may be written in at least one programming language or any combination thereof, including object oriented programming languages such as Java, Smalltalk, C ++, Python, and conventional procedural programming languages, such as "C" programming language or similar programming languages. The program code may execute entirely on the user's computer, partly on the user's computer, as a stand-alone software package, partly on the user's computer and partly on a remote computer or entirely on the remote computer or server. In the scenario associated with the remote computer, the remote computer may be connected to the user's computer through any type of networks, including a Local Area Network (LAN) or a Wide Area Network (WAN), or the connection may be made to an external computer (for example, through the Internet using an Internet service provider).

The flowchart and block diagrams in the figures illustrate the architecture, functionality, and operation of possible implementations of systems, methods and computer program products according to various embodiments of the present disclosure. In this regard, each block in the flowchart or block diagrams may represent a module, segment, or portion of code, which includes one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

The units described in this disclosure may be implemented by software or hardware. The described units may also be provided in a processor, which may be described as: a processor includes a scan data acquisition unit, a setting unit, a processing unit, and a target identification unit. The names of the units do not constitute a limitation on the units themselves in some way.

As another aspect, the present disclosure also provides a computer-readable medium, which may be contained in the device described in the above embodiments; or may be separate and not assembled into the device. The computer readable medium carries at least one program which, when executed by the device, causes the device to: acquire scan data of a subject, the scan data including data obtained by magnetic resonance imaging of the subject's brain; the scan data includes a BOLD signal sequence corresponding to each voxel in a preset rank number of voxels; determine at least two regions of interest, ROIs, of the subject from the scan data; determine ROI corresponding relationship of disease types of the subjects according to the disease types of the subjects; determine at least one disease ROI and at least one target ROI of the subject in the at least two ROIs according to the ROI corresponding relationship; determine a connectivity of voxels in the target ROI with each disease ROI in the at least one disease ROI; and determine the positions of the voxels with the connectivity meeting the preset target connectivity condition in the target ROI as the targets.

The foregoing description is only exemplary of the preferred embodiments of the disclosure and is illustrative of the principles of the technology employed. It will be appreciated by those skilled in the art that the scope of the invention in the present disclosure is not limited to the technical solutions defined by the specific combination of the above-mentioned technical features, but also encompasses other technical solutions in which any combination of the above-mentioned features or their equivalents is made without departing from the spirit of the disclosure. For example, such other technical solutions may be defined by the above features and the technical features disclosed in the present disclosure (but not limited to) having similar functions are replaced with each other.

The technical solutions described in the embodiments of the present disclosure can be arbitrarily combined without conflict.

The above description is only for the specific embodiments of the present disclosure, but the protection scope of the present disclosure is not limited thereto, and any person skilled in the art can easily think of the changes or substitutions within the technical scope of the present disclosure, and shall cover the scope of the present disclosure. Therefore, the protection scope of the present disclosure shall be subject to the protection scope of the appended claims.

**Claims**

1. A target identification method, comprising:

   acquiring scan data of a subject, the scan data comprising data obtained from magnetic resonance imaging of the subject's brain;
   determining at least two regions of interest of the subject from the scan data;
   determining corresponding relationship of regions of interest of a disease type of the subject according to the disease type of the subject;
   determining at least one disease region of interest and at least one target region of interest in the at least two regions of interest according to the corresponding relationship of the regions of interest;
determining a connectivity of voxels in the target region of interest with each of the at least one disease region of interest; and
determining positions of the voxels with the connectivities meeting a preset target connectivity condition in the target region of interest as the targets.

2. The method according to claim 1, wherein the determining the connectivity between voxels in the target region of interest and each of the at least one disease region of interest comprises:
determining connectivity coefficients of voxels in the target region of interest with each disease region of interest in the at least one disease region of interest as the connectivity between corresponding voxels in the target region of interest and a corresponding disease region of interest in the at least one disease region of interest.

3. The method according to claim 1, wherein the determining positions of the voxels with the connectivity meeting the preset target connectivity condition in the target region of interest as the targets comprises:
determining the positions of the voxels with absolute values of the connectivity greater than a preset target connectivity threshold in the target region of interest as the targets.

4. The method according to claim 1, wherein the determining positions of the voxels with the connectivity meeting the preset target connectivity condition in the target region of interest as the targets comprises:

sorting voxels in the target region of interest in descending order by the absolute values of the connectivity to obtain a voxel sequence; and
determining positions of voxels with rankings from a first one to a preset rank number in the voxel sequence as the targets.

5. The method according to claim 4, wherein the preset rank number comprises a product of the total number of voxels in the target region of interest and a preset proportion value.

6. The method according to claim 1, wherein the determining at least two regions of interest of the subject from the scan data comprises:

determining the connectivity between every two voxels in the scan data to produce a brain connectivity matrix corresponding to the scan data; and
forming the at least two regions of interest based on a brain area template of a standard brain and the brain connectivity matrix.

7. The method according to claim 1, wherein the determining at least two regions of interest of the subject from the scan data comprises:

determining the connectivity between every two voxels in the scan data;
dividing the brain anatomy of the subject corresponding to the scan data into a plurality of large regions , and subdividing each of the plurality of large regions into a plurality of brain areas, wherein each of the plurality of brain areas comprises at least one voxel; and
combining brain areas with the connectivity of voxels among the plurality of brain areas higher than a preset brain area voxel connectivity threshold and thus forming the at least two regions of interest.

8. The method according to claim 1, wherein the magnetic resonance imaging comprises: structural magnetic resonance imaging, and/or task state functional magnetic resonance imaging, and/or resting state functional magnetic resonance imaging.

9. A target identification device, comprising:

a data acquisition unit configured to acquire scan data of a subject, the scan data comprising data obtained from magnetic resonance imaging of the subject's brain;
a processing unit configured to determine at least two regions of interest of the subject from the scan data;
a setting unit configured to determine corresponding relationship of region of interest of a disease type of the subject according to the disease type and to determine at least one disease region of interest and at least one target region of interest in the at least two regions of interest according to the corresponding relationship of region of interest; and
a target identification unit,
wherein the processing unit is further configured to determine a connectivity of voxels in a target region of interest with each of the at least one disease region of interest; and
the target identification unit is configured to determine voxels in the target region of interest with the connectivity meeting a preset target connectivity condition, as the targets.

10. An electronic apparatus, comprising:

at least one processor; and
a storage device having at least one program stored thereon,
wherein the at least one program, when executed by the at least one processor, causes the at least one processor to execute the method according to any one of claims 1 to 8.

11. A computer readable storage medium having a computer program stored thereon, wherein the computer program, when executed by at least one processor, execute the method according to any one of claims 1 to 8.

12. A neuromodulation apparatus configured to make neuromodulation on a target of a subject in accordance with a preset neuromodulation solution; wherein the target is determined by the method according to any one of claims 1 to 8.

13. The apparatus according to claim 12, wherein the preset neuromodulation solution comprises at least one of:

deep brain electrical stimulation;
transcranial electrical stimulation;
electroconvulsive therapy;
electrical stimulation based on cortical brain electrodes;
transcranial magnetic stimulation;
focused ultrasound neuromodulation;
magnetic resonance guided high-intensity focused ultrasound therapy neuromodulation; and
photobiomodulation therapy.

FIG. 1

**200**

201

Acquiring scan data of a subject

202

Determining at least two ROIs of the subject from the scan data

203

Determining ROI corresponding relationship of disease type of the subject according to the disease type of the subject

204

Determining at least one disease ROI and at least one target ROI in the at least two ROIs according to the ROI corresponding relationship

205

Determining a connectivity of voxels in the target ROI with each of the at least one disease ROI

206

Determining the positions of the voxel with the connectivity meeting the preset target connectivity condition in the target ROI as the target

FIG. 2

202

202a1

Determining the connectivity between every two voxels in the scan data to produce a brain connectivity matrix corresponding to the scan data

202a2

Forming the at least two ROIs on a basis of the standard brain ROI template and the brain connectivity matrix

FIG. 3

202

202b1

Determining the connectivity between every two voxels in the scan data

202b2

Dividing the brain anatomy of the subject corresponding to the scan data into a plurality of large regions, and subdividing each of the plurality of large regions into a plurality of brain areas, in which each of the plurality of brain areas includes at least one voxel

202b3

Combining brain areas which have the voxel connectivity among brain areas higher than a preset brain area voxel connectivity threshold in the plurality of brain areas, thereby forming at least two ROIs

FIG. 4

FIG. 5

FIG. 6

FIG. 7

FIG. 8

## INTERNATIONAL SEARCH REPORT

| International application No. |
|---|
| **PCT/CN2022/101590** |

| A. | CLASSIFICATION OF SUBJECT MATTER |
|---|---|

A61B 5/055(2006.01)i;　A61N 1/00(2006.01)i;　A61N 2/00(2006.01)i

According to International Patent Classification (IPC) or to both national classification and IPC

| B. | FIELDS SEARCHED |
|---|---|

Minimum documentation searched (classification system followed by classification symbols)

A61B5 A61N1 A61N2

Documentation searched other than minimum documentation to the extent that such documents are included in the fields searched

Electronic data base consulted during the international search (name of data base and, where practicable, search terms used)

CNABS, CNTXT: 磁共振, 感兴趣, 区域, 选择, 选取, 选定, 靶点, 靶位, 靶区, 靶标, 连接, 体素, 刺激; VEN, USTXT, EPTXT, WOTXT: MR, interesting, region, select, target, connet, voxel, stimulate.

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 113367681 A (BEIJING YINHE FANGYUAN TECHNOLOGY CO., LTD.) 10 September 2021 (2021-09-10) description, paragraphs 3-57 | 1-13 |
| X | US 2015119689 A1 (BETH ISRAEL DEACONESS MEDICAL CENTER, INC.) 30 April 2015 (2015-04-30) description, paragraphs 39-100, and figures 1-23 | 1-13 |
| X | US 2021170180 A1 (DOSENBACH, N. et al.) 10 June 2021 (2021-06-10) description, paragraphs 49-72, and figures 1-24 | 1-13 |
| X | CN 112546446 A (SIR RUN RUN SHAW HOSPITAL, ZHEJIANG UNIVERSITY SCHOOL OF MEDICINE) 26 March 2021 (2021-03-26) description, paragraphs 17-22, and figure 1 | 1-13 |
| X | CN 111407276 A (HANGZHOU NORMAL UNIVERSITY) 14 July 2020 (2020-07-14) description, paragraphs 31-46, and figures 1-2 | 1-13 |
| A | US 2016260224 A1 (LONDON HEALTH SCIENCES CENTRE RESEARCH INC.) 08 September 2016 (2016-09-08) entire document | 1-13 |

☑ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 September 2022** | **14 September 2022** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/101590**

| C. | DOCUMENTS CONSIDERED TO BE RELEVANT | |
|---|---|---|
| Category* | Citation of document, with indication, where appropriate, of the relevant passages | Relevant to claim No. |
| A | CN 109480841 A (FUDAN UNIVERSITY) 19 March 2019 (2019-03-19)<br>entire document | 1-13 |
| A | CN 108355250 A (UNIVERSITY OF ELECTRONIC SCIENCE AND TECHNOLOGY OF CHINA) 03 August 2018 (2018-08-03)<br>entire document | 1-13 |
| A | WO 2009044270 A2 (NEURONIX LTD.) 09 April 2009 (2009-04-09)<br>entire document | 1-13 |

Form PCT/ISA/210 (second sheet) (January 2015)

**INTERNATIONAL SEARCH REPORT**
Information on patent family members

International application No.

**PCT/CN2022/101590**

| Patent document cited in search report | | | Publication date (day/month/year) | Patent family member(s) | | | Publication date (day/month/year) |
|---|---|---|---|---|---|---|---|
| CN | 113367681 | A | 10 September 2021 | None | | | |
| US | 2015119689 | A1 | 30 April 2015 | WO | 2013172981 | A1 | 21 November 2013 |
| | | | | US | 10137307 | B2 | 27 November 2018 |
| US | 2021170180 | A1 | 10 June 2021 | US | 2021333343 | A1 | 28 October 2021 |
| CN | 112546446 | A | 26 March 2021 | None | | | |
| CN | 111407276 | A | 14 July 2020 | None | | | |
| US | 2016260224 | A1 | 08 September 2016 | CA | 2929014 | A1 | 07 May 2015 |
| | | | | WO | 2015061882 | A1 | 07 May 2015 |
| | | | | US | 10198825 | B2 | 05 February 2019 |
| CN | 109480841 | A | 19 March 2019 | None | | | |
| CN | 108355250 | A | 03 August 2018 | CN | 108355250 | B | 27 August 2021 |
| WO | 2009044270 | A2 | 09 April 2009 | None | | | |

Form PCT/ISA/210 (patent family annex) (January 2015)